# EUROPEAN PATENT APPLICATION

(11) **EP 3 045 546 A1**
(43) Date of publication of application: **20.07.2016**
(21) Application number: 16159330.6
(22) Date of filing: 09.07.2010
(51) Int. Cl.: C12Q 1/68

(54) **METHODS OF CLASSIFYING BIOLOGICAL SAMPLES FOR PREDICTING RESPONSE TO TYROSINE KINASE INHIBITOR TREATMENT**

(30) Priority: 09.07.2009 US 224281 P
(62) Divisional of application: 10734406.1
(71) Applicant: Abbott Molecular Inc., Des Plaines, IL 60018 (US)
(72) Inventor: COON, John, Oak Park, IL 60302 (US); MORRISON, Larry, Lombard, IL 60148 (US)
(74) Representative: Modiano, Micaela Nadia

(57) **Abstract**

Gene copy numbers of signaling components downstream of EGFR identify non- small cell lung cancer (NSCLC) patients with poor outcomes on 2nd/3rd line gefitinib therapy.

## Description

### CROSS-REFERENCE TO RELATED APPLICATIONS

The present application claims priority to U.S. Provisional application number 61/224,281 filed July 9, 2009, the entirety of each of which is herein incorporated by reference.

### BACKGROUND

EGFR mutations in exons 19 and 21 and high EGFR copy number have been associated with increased sensitivity to EGFR tyrosine kinase inhibitors (TKI's) in NSCLC patients, while KRAS mutations, MET expression, and EGFR T790M mutation have been linked to resistance. In preclinical models, EGFR downstream markers have been important in EGFR sensitivity or resistance. In this study we have examined the impact of abnormal PTEN and PIK3CA gene copy numbers on the outcomes of patients treated with gefitinib. Methods: Formalin-fixed paraffin embedded tissues and cell pellets from 81 gefitinibtreated NSCLC patients were analyzed by fluorescence in situ hybridization (FISH) with probes specific for EGFR, PTEN, PIK3CA, and centromeres (cen) 3, 7, and 10, and 72 specimens yielded results for all 6 probes. Patients were previously treated with at least one chemotherapy regimen or were considered ineligible for chemotherapy. FISH signals were enumerated in ≥40 cells per specimen to obtain copy numbers for each locus. Various classifiers were derived from the genomic copy numbers and a range of cutoff values were applied to classify patients by objective response (OR), progression free survival (PFS), and overall survival (OS). EGFR. mutation status (exons 19 & 21) was obtained for 55 of the specimens.
Results: Loss of PTEN (≥20% of cells with <2 copies of PTEN) and low cen7 copy number (average cen7 copies/cell <4) were each significantly associated with lower OS, and high PIK3CA copy number (≥40% of cells with >2 copies of PIK3CA) and low EGFR copy number (<75% cells with >2 copies of EGFR) showed trends toward lower OS. Combined loss of PTEN and high PIK3CA copy number, or 3-way combinations of high PIK3CA copy number, loss of PTEN, and either low EGFR copy number or low cen7 copy number, were significantly associated with lower OS and lower PFS. Twenty two patients (31%) had tumors with high PIK3CA copy number, loss of PTEN, and low cen7 copy number, and these patients exhibited median OS of 132 d compared to 373 d in the other 50 patients (p<0.0001 Kaplan-Meier analysis, log rank test), median PFS of 62 d compared to 128 d (p=0.0007), and 0% OR compared to 22% (p=0.015, 2-tail Fischer's exact test). In the 37 patients that did not harbor an EGFR mutation, the classifier was still significant (median OS: 128 vs 380 d, p<0.0001; median PFS: 62 vs 102 d, p=0.019). In multivariate analysis, including PIK3CA, PATEN, and cen7 FISH status, gender, histology, and EGFR mutation status, all 3 FISH parameters remained significantly associated with OS. Conclusions: These data indicate that gefitinib is relatively ineffective in tumors with relatively high PTEN loss and PIK3CA gain, and low levels of EGFR or cen7, in either the full study group or the subgroup without EGFR mutations. If confirmed in subsequent studies, alternative treatments, potentially including agents selected for combination with EGFR TKI's, should be considered for patients whose tumors have this profile.

### DESCRIPTION

The method of the invention is an clinical laboratory assay used to classify cancer or suspected cancer patient tissue samples into separate copy number profiles, indicative of response to tyrosine kinase inhibitors, such as gefitinib, which is a small molecule inhibitor of the EGFR receptor protein. The invention is the use of an in situ hybridization assay, preferably based on fluorescence in situ hybridization (FISH), using chromosomal hybridization probes to determine copy number at two, or three human chromosome loci: 10q23.3, 3q26.3 and chromosome 7, preferably either its centromere or at locus 7p12. Determination of the copy number at these loci in cells in a lung tissue sample can be used to classify the sample as having a copy number profile indicative of either resistance or response to a tyrosine kinase inhibitor. In the preferred embodiments, tissue sample determined as having relative loss of 10q23.3, relative gain of 3q26.3 and relative low copy number of chromosome 7, compared to normal copy numbers at these loci, is classified as having a copy number profile of 10q23.3 loss, 3q26.3 gain and low copy number chromosome 7, which marks resistance to these inhibitors. Where the sample is classified as having any other copy number profile for these loci, the profile marks sensitivity or response to these inhibitors. In another preferred embodiment, the copy number profile is of the two loci 10q23.3 and 3q26.3. The invention is believed useful with all forms of tyrosine kinase inhibitors of genes in the EGFR pathway.
The invention is also notable because the copy number profile classification is independent of EGFR mutation status in the tissue sample.

The invention is also advantageous because the copy number profile was statistically significant over relatively wide ranges of percent assessable cells in the samples as having the particular chromosomal abnormality, ie 10q23.3 loss or 3q26.3 gain. For the 10q23.3 loss, the range of cells is about 13 to about 35% of the assessed cells showing relative loss, preferably about 20%. For the 3q26.3 gain, the range of cells is about 20 to about 62.5% of the assessed cells showing relative gain, preferably about 40%. The chromosome 7 copy number measurement showed robust statistical results for chromosome 7 copy numbers measured in the range of about 2.7 to about 5.1 of the assessed cells, preferably about 4 copies per cell.

The in situ hybridization chromosomal probes suitable for use in this invention include the EGFR and chromosome 7 centromere probes described in commonly assigned, co-pending U.S. application, "Diagnostic methods for determining treatment", L.E. Morrison and J.S. Coon, filed May 8, 2007, published as US 20070275403 A1, incorporated herein by reference in its entirety. The in situ hybridization chromosomal probes suitable for use to determine copy number at the P1K3CA locu can be manufactured using the methods described in co-pending, commonly-assigned U.S. application, S.N. 12/268,797, "Prognostic Test for Early Stage Non Small Cell Lung Cancer", L.E. Morrison and J.S. Coon, filed November 11, 2008, and incorporated herein in its entirety, herein "Morrison II", starting with clones containing human insert DNA containing nucleic acid sequences at the loci of PIK3CA. The Morrison II application also describes a suitable probe, designed to hybridize to the PTEN locus, for use herein. Probes useful herein are not limited to DNA based probes, but include peptide nucleic acid based probes. The in situ hybridization method used to determine the copy number profiles of this invention is described in our co-pending US applications. The copy numbers at each chromosome locus are determining preferably by standard FISH image analysis, either manual or using digital imaging software based methods.

The present invention includes the following embodiments indicated as items 1-22
1. A method for classifying a biological sample with respect to treatment with an inhibitor of the tyrosine kinase activity of EGFR or an agent that functions similarly to tyrosine kinase inhibitors, the method comprising:
   (a) obtaining a biological sample from a human patient;
   (b) contacting the biological sample with a set of chromosomal hybridization probes under conditions sufficient to enable hybridization of the probes to chromosomes in the sample if any, wherein (i) one probe is designed to detect copy number of human Chromosome 7 in cells in the sample, (ii) one probe is designed to detect copy number of human Chromosome locus 10q23.3 in cells in the sample and (iii) one probe is designed to detect copy number of human Chromosome locus 3q26.3 in cells in the sample;
   (c) determining copy number for each of human Chromosome 7, human Chromosome locus 10q23.3 and human chromosome locus 3q26.3; and
   (d) classifying the sample based on results of step (c), as either having a copy number profile of 10q23.3 loss, 3q26.3 gain and chromosome 7 loss, relative to normal copy number for each, or having any other copy number profile.
2. The method of item 1 wherein the biological sample is a lung biopsy sample.
3. The method of item 1 wherein the biological sample is from a patient previously diagnosed as having non-small cell lung cancer.
4. The method of item 1 wherein one of the probes is designed to hybridize to at least a one nucleic acid sequence present in a PTEN gene at locus 10q23.3.
5. The method of item 1 wherein one of the probes is designed to hybridize to at least one nucleic acid sequence present in a PIK3CA gene at locus 3q26.3.
6. The method of item. 1 wherein one of the probes is designed to hybridize to at least one nucleic acid sequence in human Chromosome 7 centromere.
7. The method of item 1 wherein one of the probes is designed to hybridize to at least a one nucleic acid sequence present in a PTEN gene at locus 10q23.3, one of the probes is designed to hybridize to at least one nucleic acid sequence present in a PIK3CA gene at locus 3q26.3, and one of the probes is designed to hybridize to at least one nucleic acid sequence in human Chromosome 7 centromere.
8. The method of item 1 wherein each of the probes is fluorescently labeled and are detectable simultaneously.
9. The method of item 1 wherein one of the probes is designed to hybridize to at least one nucleic acid sequence in human EGFR gene at locus 7p12.
10. The method of item 9 wherein one of the probes is designed to hybridize to at least a one nucleic acid sequence present in a PTEN gene at locus 10q23.3, and one of the probes is designed to hybridize to at least one nucleic acid sequence present in a PIK3CA gene at locus 3q26.3.
11. The method of item 1 wherein the determining copy number step (c) comprises assessing at least 40 cells in the sample.
12. The method of item 1 wherein copy number profile of 10q23.3 loss comprises determination that ≥20% of cells have less than 2 copies of PTEN.
13. The method ofitem 1 wherein copy number profile of 10q23.3 loss comprises determination that average Chromosome 7 copy number in assessable cells in the sample is less than 4.
14. The method of item 1 wherein copy number profile of 10q23.3 loss comprises determination that ≥40% of assessable cells in the sample have more than 2 copies of PIK3CA.
15. A method for classifying a biological sample with respect to treatment with an inhibitor of the tyrosine kinase activity of EGFR or an agent that functions similarly to tyrosine kinase inhibitors, the method comprising:
   (a) obtaining a biological sample from a human patient;
   (b) contacting the biological sample with a set of chromosomal hybridization probes under conditions sufficient to enable hybridization of the probes to chromosomes in the sample if any, wherein (i) one probe is designed to detect copy number of human Chromosome locus 10q23.3 in cells in the sample and (ii) one probe is designed to detect copy number of human Chromosome locus 3q26.3 in cells in the sample;
   (c) determining copy number for each of human Chromosome locus 10q23.3 and human chromosome locus 3q26.3; and
   (d) classifying the sample based on results of step (c), as either having a copy number profile of 10q23.3 loss and 3q26.3 gain, relative to normal copy number for each, or having any other copy number profile.
16. The method of item 15 wherein the biological sample is a lung biopsy sample.
17. The method of item 15 wherein the biological sample is from a patient previously diagnosed as having non-small cell lung cancer.
18. The method of item 15 wherein one of the probes is designed to hybridize to at least a one nucleic acid sequence present in a PTEN gene at locus 10q23.3.
19. The method of item 15 wherein one of the probes is designed to hybridize to at least one nucleic acid sequence present in a PIK3CA gene at locus 3q26.3.
20. The method of item 15 wherein one of the probes is designed to hybridize to at least a one nucleic acid sequence present in a PTEN gene at locus 10q23.3, and one of the probes is designed to hybridize to at least one nucleic acid sequence present in a PIK3CA gene at locus 3q26.3.
21. The method of item 15 wherein copy number profile of 10q23.3 loss comprises determination that ≥20% of cells have less than 2 copies of PTEN.
22. The method of item 15 wherein copy number profile of 10q23.3 loss comprises determination that ≥40% of assessable cells in the sample have more than 2 copies of PIK3CA.

## Claims

1. A method for determining a copy number profile, the method comprising: (a) contacting a biological sample obtained from a human patient with a set of chromosomal hybridization probes under conditions sufficient to enable hybridization of the probes to chromosomes in said sample wherein (i) one probe is designed to detect a copy number of human Chromosome locus 10q23.3 in cells in said sample and (ii) one probe is designed to detect a copy number of human Chromosome locus 3q26.3 in cells in said sample wherein each of said probes is fluorescently labeled and wherein each of said labeled probes are detectable simultaneously; and (b) using in situ hybridization to determine a copy number for each of said human Chromosome locus 10q23.3 and said human Chromosome locus 3q26.3 to determine said copy number profile.

2. The method of claim 1 further comprising: in step (a) contacting said biological sample with (iii) a probe designed to detect a copy number of human Chromosome 7 in cells in said sample wherein said probe is fluorescently labeled wherein each of said labeled probes are detectable simultaneously; and in step (b) determining a copy number for Chromosome 7.

3. The method of claim 2 wherein one of the probes is designed to hybridize to at least one nucleic acid sequence in human Chromosome 7 centromere.

4. The method of claim 2 wherein one of the probes is designed to hybridize to at least one nucleic acid sequence present in a PTEN gene at locus 10q23.3, one of the probes is designed to hybridize to at least one nucleic acid sequence present in a PIK3CA gene at locus 3q26.3, and one of the probes is designed to hybridize to at least one nucleic acid sequence in human Chromosome 7 centromere.

5. The method of claim 2 wherein one of the probes is designed to hybridize to at least one nucleic acid sequence in human EGFR gene at locus 7p12.

6. The method of claim 5 wherein one of the probes is designed to hybridize to at least one nucleic acid sequence present in a PTEN gene at locus 10q23.3, and one of the probes is designed to hybridize to at least one nucleic acid sequence present in a PIK3CA gene at locus 3q26.3.

7. The method of claim 2 wherein the determining copy number step (c) comprises assessing at least 40 cells in the sample.

8. The method of claim 2 wherein copy number profile of 10q23.3 loss comprises determination that average Chromosome 7 copy number in assessable cells in the sample is less than 4.

9. The method of claim 1 or 2 wherein the biological sample is a lung biopsy sample.

10. The method of claim 1 or 2 wherein the biological sample is from a patient previously diagnosed as having non-small cell lung cancer.

11. The method of claim 1 or 2 wherein one of the probes is designed to hybridize to at least one nucleic acid sequence present in a PTEN gene at locus 10q23.3.

12. The method of claim 1 wherein one of the probes is designed to hybridize to at least one nucleic acid sequence present in a PTEN gene at locus 10q23.3, and one of the probes is designed to hybridize to at least one nucleic acid sequence present in a PIK3CA gene at locus 3q26.3.

13. The method of claim 1 or 2 wherein copy number profile of 10q23.3 loss comprises determination that ≥20% of assessable cells in the sample have less than 2 copies of PTEN.

14. The method of claim 1 or 2 wherein copy number profile of 10q23.3 loss comprises determination that ≥40% of assessable cells in the sample have more than 2 copies of PIK3CA.
